# EUROPÄISCHE PATENTSCHRIFT

(11) **EP 2 699 555 B1**
(45) Veröffentlichungstag und Bekanntmachung des Hinweises auf die Patenterteilung: **07.10.2015**
(21) Anmeldenummer: 12724534.8
(22) Anmeldetag: 16.04.2012
(51) Int. Cl.: C07D 251/54, C08K 5/3492

(54) **MELAMINTHIOSULFAT**
MELAMINE THIOSULFATE
THIOSULFATE DE MÉLAMINE

(30) Priorität: 18.04.2011 AT 5472011
(43) Veröffentlichungstag der Anmeldung: 26.02.2014
(73) Patentinhaber: Metadynea Austria GmbH, 3500 Krems (AT)
(72) Erfinder: ZICH, Thomas, A-4020 Linz (AT); FREIDL, Fritz Johann, A-3500 Krems (AT); MEHOFER, Bernadette, A-3500 Krems (AT); HÖNEL, Michael, A-8010 Graz (AT)
(74) Vertreter: Verbart, Jeannette J.M.
(86) Internationale Anmeldenummer: PCT/AT2012/050050
(87) Internationale Veröffentlichungsnummer: WO 2012/142642

(56) Entgegenhaltungen:
- EP-A1- 2 159 265
- EP-A2- 1 081 183
- WO-A1-2011/000019

## Beschreibung

Die vorliegende Erfindung betrifft Melamin(ium)thiosulfat und dessen Verwendung als Flammschutzmittel.

Von elementarem Schwefel und schwefelhältigen Verbindungen ist bekannt, dass sie Wirkung als Flammschutzmittel zeigen - entweder als alleiniges Flammschutzmittel oder als Synergist in Kombination mit anderen bekannten Flammschutzmitteln, z.B. mit Derivaten von 9,10-Dihydro-9-oxa-10-phosphaphenanthren-10-on bzw. -oxid (DOPO), wie dies die Erfinder des vorliegenden Anmeldungsgegenstands herausgefunden haben; siehe unter anderem WO 2011/000019 A1. Um eine solche synergistische Wirkung zu entfalten, sollte eine schwefelhaltige Verbindung vorzugsweise eine oder mehrere S-S-Bindungen aufweisen, z.B. Thiosulfate. Auch Melaminsalze als DOPO-Derivate können flammhemmende Wirkung besitzen, wie dies in der WO 2011/000019 A1 der Erfinder offenbart wird. Dieser Synergismus hat sich darüber hinaus als äußerst substanzspezifisch erwiesen, d.h. nicht jede schwefelhaltige, als flammschutzfördernd bekannte Verbindung wirkt in gleichem Ausmaß synergistisch, wenn überhaupt.

Ein schwerer Nachteil zahlreicher schwefelhaltiger Verbindungen besteht jedoch im mehr oder weniger stark ausgeprägten Geruch, weswegen diese Verbindungen für eine Vielzahl von Anwendungen nicht in Frage kommen.

Ziel der Erfindung war daher die Herstellung neuer Schwefelverbindungen und Überprüfung, ob diese ebenfalls flammhemmende Wirkung zeigen.

### OFFENBARUNG DER ERFINDUNG

Dieses Ziel erreicht die Erfindung in einem ersten Aspekt durch Bereitstellung von Melaminthiosulfat oder Bis[(2,4,6-triamino-1,3,5-triazinium)thiosulfat:

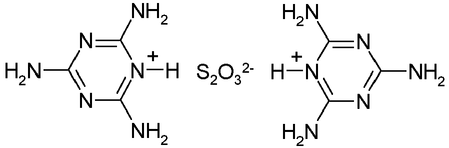

Überraschenderweise hat sich Melaminthiosulfat in den Tests auf seine flammhemmende Wirkung - im Gegensatz zu anderen Thiosulfaten, wie z.B. Ammoniumthiosulfat - als praktisch geruchlos erwiesen.

In einem zweiten Aspekt umfasst die Erfindung ein Verfahren zur Herstellung von Melaminthiosulfat aus Melamin das dadurch gekennzeichnet ist, dass Melamin in Gegenwart einer Säure, vorzugsweise einer starken Säure wie Salzsäure oder Phosphorsäure, mit Natriumthiosulfat umgesetzt wird. Durch eine derartige Umsalzung ist Melaminthiosulfat in guten Ausbeuten und in hoher Reinheit erhältlich.

In den Anwendungsbeispielen hat sich gezeigt, dass zwar Melaminthiosulfat alleine - zumindest in praxistauglichen Konzentrationen - kaum ausreichende Flammschutzwirkung zeigt, dafür aber eine starke Wirkung als Synergist in Kombination mit bekannten Flammschutzmitteln entfaltet. Somit betrifft die Erfindung in einem dritten Aspekt die Verwendung von Melaminthiosulfat als Flammschutzmittel, vorzugsweise als Synergist in Kombination mit bekannten Flammschutzmitteln, z.B. als Bestandteil einer Flammschutzzusammensetzung.

In einem vierten Aspekt betrifft die Erfindung eine solche Flammschutzzusammensetzung, die zumindest ein bekanntes Flammschutzmittel und gegebenenfalls ein(en) oder mehrere übliche Additive, Hilfsstoffe und/oder Synergisten umfasst, in der zusätzlich Melaminthiosulfat als Synergist enthalten ist.

Nachstehend werden drei Synthesewege zur Herstellung von Melaminthiosulfat beschrieben und die so hergestellte neue Verbindung auf ihre flammhemmende Wirkung getestet. Die Schmelzpunkte bzw. Zersetzungspunkte wurden auf einem Kofler-Heiztischmikroskop gemessen und sind unkorrigiert.

### BEISPIELE

### Beispiele 1 bis 3 - Herstellung vom Melaminthiosulfat

### Beispiel 1

In einem Mehrhalskolben, ausgestattet mit einem Rührwerk, Rückflusskühler und Thermometer, wurden 1218,7 g destilliertes Wasser mit 147,8 g konz. Salzsäure (37%ig) und 189,1 g Melamin vermischt. Die Suspension wurde auf Rückflusstemperatur erhitzt. Nachdem eine klare Lösung vorlag, wurde der Kolbeninhalt auf 96 °C abgekühlt und mit 348,6 g einer 34%igen Natriumthiosulfatlösung versetzt. Dabei trat eine Fällungsreaktion ein. Der Niederschlag wurde unter Rühren auf Raumtemperatur abgekühlt, abfiltriert und intensiv mit destilliertem Wasser nachgewaschen. Die Trocknung des Filterrückstands erfolgte bei 110 °C. Die Ausbeute betrug 265,1 g [96,5 % d. Th.] eines weißen, kristallinen Feststoffs.
Fp.: 178-180 °C (Zers.)
Elementaranalyse C₆H₁₄N₁₂O₃S₂ (M: 366,38 g/mol):
ber. C: 19,67 %; H: 3,85 %; N: 45,88 %; O: 13,10 %; S: 17,50 %
gef. C: 19,8 %; H: 4,0 %; N: 45,6 %; O: 13,5 %; S: 17,2 %

### Beispiel 2

In einem Mehrhalskolben, ausgestattet mit einem Rührwerk, Rückflusskühler und Thermometer, wurden 1200,0 g destilliertes Wasser mit 252,2 g Melamin und 158,1 g Natriumthiosulfat versetzt. Die Suspension wurde auf 95 °C erhitzt. Anschließend wurden mit einer Dosiergeschwindigkeit von 0,9 g/min 197,1 g konz. Salzsäure (37%ig) zugetropft. Danach wurde die Reaktionsmasse auf unter Rühren auf Raumtemperatur abgekühlt, der Niederschlag abfiltriert und mit destillierten Wasser nachgewaschen. Der Filterkuchen wurde nochmals in 1100 g destilliertem Wasser aufgenommen, intensiv gerührt und abfiltriert. Die Trocknung des Filterrückstands erfolgte bei 110 °C. Die Ausbeute betrug 356,8 g [97,4 % d. Th.] eines weißen, kristallinen Feststoffs, dessen Daten mit jenen aus Beispiel 1 im Wesentlichen übereinstimmten.

### Beispiel 3

In einem Mehrhalskolben, ausgestattet mit einem Rührwerk, Rückflusskühler und Thermometer, wurden 1130 g destilliertes Wasser, 252,2 g Melamin und 158,1 g Natriumthiosulfat vermischt und auf 90 °C erhitzt. Innerhalb von 1,5 Stunden wurden zur vorgelegten Suspension 174,2 g 37,5%ige Phosphorsäure bei 90-93 °C zugetropft. Danach wurde der Niederschlag bei laufenden Rührwerk auf Raumtemperatur abgekühlt und über ein Blaubandfilter abfiltriert. Der Filterkuchen wurde mit Wasser nachgewaschen und anschließend bei 110 °C getrocknet. Die Ausbeute betrug 328,5 g [89 % d. Th.] eines weißen, kristallinen Feststoffs, dessen Daten mit jenen aus Beispiel 1 im Wesentlichen übereinstimmten.

### Vergleichsbeispiele 1 bis 8 und Beispiele 4 bis 7 - Anwendungsbeispiele für die Flammschutzausrüstung von Kunststoffen

Zur Untersuchung der Nachbrenndauer (in s) bei Beflammung wurden für Polystyrol gemäß UL94 Probenkörper mit 70 x 13 x 4 mm hergestellt. Kürzere Brenndauer-Werte stehen folglich für eine bessere Brandschutzwirkung.

Aus Polystyrol-Granulat (M_{w}: ca. 192.000 g/mol, Tg: ca. 94 °C) wurden wie folgt Probenkörper geformt. Das Granulat wurde zu einem Pulver vermahlen und in einem Mörser mit den jeweiligen Zusätzen, d.h. Flammschutzmittel und/oder Synergist, vermischt (in Vergleichsbeispiel 1 ohne jeden Zusatz). Je 12 g dieser Feststoff-Mischungen wurden in Aluminiumtiegel eingewogen, die anschließend in einen vorgeheizten Trockenschrank gestellt und so lange bei der jeweils erforderlichen Temperatur darin belassen wurden, bis das Pulver zu kompakten Platten verschmolzen war. Die dafür erforderliche Temperatur ist von der Zusammensetzung der jeweiligen Mischung abhängig und lag bei den untersuchten Probekörpern zwischen 180 und 190 °C, und der Schmelzvorgang war nach 12 bis 15 min beendet, wie dies in der nachfolgenden Tabelle angegeben ist. Nach dem Abkühlen wurden die Platten aus den Aluminiumtiegeln entnommen und zu Prüfkörpern für die Flammschutztests zersägt.

Als Flammschutzmittel bzw. Synergisten wurden neben Melaminthiosulfat (MelTS) elementarer Schwefel, Vultac TB7, ein p-t-Butylphenoldisulfid-Polymer der Fa. Arkema, Ammoniumthiosulfat ((NH₄)₂S₂O₃; ATS), DOPO-ONH₄ und DOPO-OMel (Strukturen umseitig) getestet. Die für die Probenkörper erhaltenen Ergebnisse sind in umseitiger Tabelle 1 angeführt und stellen Mittelwerte von je vier Messungen dar.

**Tabelle 1 - Polystyrolproben**

| **Beispiel** | **Flammschutzmittel** | **Gew.-%** | **Synergist** | **Gew.-%** | **Polystyrol Gew.-%** | **Verarbeitungsbedingungen** | **Brenndauer, s** |
|---|---|---|---|---|---|---|---|
| Vergl. 1 | - | - | - | - | 100,0 | 190 °C, 15 min | k.S. |
| Vergl. 2 | Schwefel | 5,0 | - | - | 95,0 | 190 °C, 15 min | 40 |
| Vergl. 3 | - | - | Vultac TB7 | 5,0 | 95,0 | 180 °C, 15 min | k.S. |
| Vergl. 4 | - | - | ATS | 4,0 | 96,0 | 180 °C, 15 min | k.S. |
| Beispiel 4 | - | - | MelTS | 8,0 | 92,0 | 180 °C, 15 min | k.S. |
| Beispiel 5 | MelTS | 30,0 | - | - | 70,0 | 180 °C, 15 min | 40 |
| Beispiel 6 | DOPO-ONH₄ | 8,0 | MelTS | 7,7 | 84,3 | 190 °C, 15 min | 5 |
| Vergl. 5 | DOPO-ONH4 | 2,5 | Vultac TB7 | 4,0 | 93,5 | 190 °C, 15 min | 5 |
| Vergl. 6 | DOPO-ONH₄ | 4,0 | ATS | 6,0 | 90,0 | 190 °C, 15 min | 10 |
| Beispiel 7 | DOPO-OMel | 5,0 | MelTS | 7,0 | 88,0 | 190 °C, 15 min | 7 |
| Vergl. 7 | DOPO-OMel | 3,0 | Vultac TB7 | 4,0 | 93,0 | 190 °C, 15 min | 5 |
| Vergl. 8 | DOPO-OMel | 5,0 | ATS | 5,0 | 90,0 | 190 °C, 15 min | 10 |

| | | | | | | | |
|---|---|---|---|---|---|---|---|
| k.S.: keine Selbstverlöschung | | | | | | | |

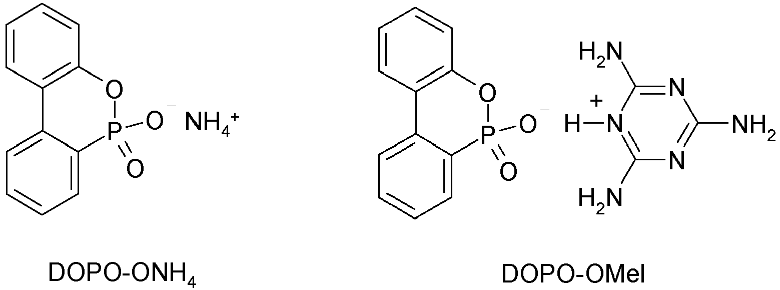

Aus der obigen Tabelle 1 geht klar hervor, dass Melaminthiosulfat alleine in praxistauglichen Konzentrationen zwar nur schwache Flammschutzwirkung aufweist, dass es aber ein ähnlich guter Synergist für bekannte Flammschutzmittel ist wie Ammoniumthiosulfat und Vultac TB7 und im Gegensatz zu diesen beiden Verbindungen bei der Einarbeitung in den Kunststoff praktisch geruchlos ist.

Somit stellt die Erfindung mit Melaminthiosulfat eine neue Substanz bereit, die sehr gut zur Flammschutzausrüstung von Kunststoffen geeignet ist.

## Patentansprüche

1. Melaminthiosulfat oder Bis(2,4,6-triamino-1,3,5-triazinium)thiosulfat:

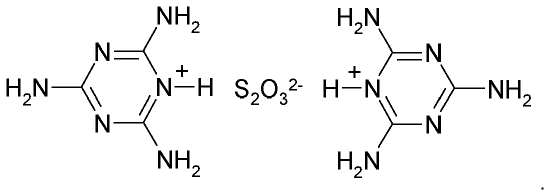

2. Verfahren zur Herstellung von Melaminthiosulfat nach Anspruch 1, **dadurch gekennzeichnet, dass** Melamin in Gegenwart einer Säure mit Natriumthiosulfat umgesetzt wird.

3. Verfahren nach Anspruch 2, **dadurch gekennzeichnet, dass** eine starke Säure wie Salzsäure oder Phosphorsäure eingesetzt wird.

4. Verwendung von Melaminthiosulfat als Flammschutzmittel.

5. Verwendung nach Anspruch 4, **dadurch gekennzeichnet, dass** Melaminthiosulfat als Synergist in Kombination mit bekannten Flammschutzmitteln eingesetzt wird.

6. Verwendung nach Anspruch 4 oder 5, **dadurch gekennzeichnet, dass** das Melaminthiosulfat als Bestandteil einer Flammschutzzusammensetzung eingesetzt wird.

7. Flammschutzzusammensetzung, umfassend zumindest ein bekanntes Flammschutzmittel und gegebenenfalls ein(en) oder mehrere übliche Additive, Hilfsstoffe und/oder Synergisten, **dadurch gekennzeichnet, dass** zusätzlich Melaminthiosulfat als Synergist enthalten ist.

## Claims

1. Melamine thiosulphate or bis(2,4,6-triamino-1,3,5-triazinium)thiosulphate:

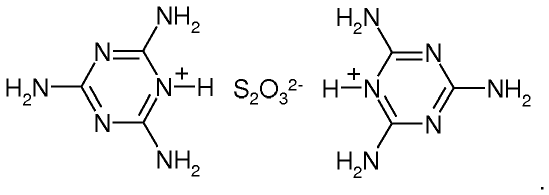

2. A method for the manufacture of melamine thiosulphate as claimed in claim 1, **characterized in that** melamine is reacted with sodium thiosulphate in the presence of an acid.

3. The method as claimed in claim 2, **characterized in that** a strong acid such as hydrochloric acid or phosphoric acid is used.

4. Use of melamine thiosulphate as a flame retardant.

5. Use as claimed in claim 4, **characterized in that** melamine thiosulphate is used as a synergist in combination with known flame retardants.

6. Use as claimed in claim 4 or claim 5, **characterized in that** the melamine thiosulphate is used as a component of a flame retardant composition.

7. A flame retardant composition comprising at least one known flame retardant and one or more customary additives, adjuvants and/or synergists if appropriate, **characterized in that** it additionally contains melamine thiosulphate as a synergist.

## Revendications

1. Thiosulfate de mélamine ou thiosulfate de bis(2,4,6-triamino-1,3,5-triazinium) :

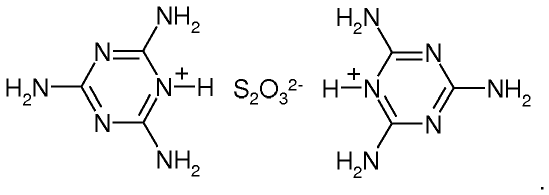

2. Procédé de préparation de thiosulfate de mélamine selon la revendication 1, **caractérisé en ce qu'**on transforme de la mélamine en présence d'un acide avec du thiosulfate de sodium.

3. Procédé selon la revendication 2, **caractérisé en ce qu'**on met en oeuvre un acide fort, comme de l'acide chlorhydrique ou de l'acide phosphorique.

4. Utilisation de thiosulfate de mélamine en tant qu'agent ignifugeant.

5. Utilisation selon la revendication 4, **caractérisée en ce qu'**on met en oeuvre du thiosulfate de mélamine en tant qu'agent synergique, en association avec des agents ignifugeants connus.

6. Utilisation selon la revendication 4 ou la revendication 5, **caractérisée en ce qu'**on met en oeuvre le thiosulfate de mélamine en tant que composant d'une composition ignifugeante.

7. Composition ignifugeante, comprenant au moins un agent ignifugeant connu et le cas échéant, un ou plusieurs additifs, agents auxiliaires et/ou agents synergiques usuels, **caractérisée en ce qu'**elle contient en supplément du thiosulfate de mélamine en tant qu'agent synergique.
